Europäisches Patentamt

European Patent Office

Office européen des brevets

(19)

(11) **EP 0 715 845 B1**

(12) **FASCICULE DE BREVET EUROPEEN**

(45) Date de publication et mention
de la délivrance du brevet:
**20.08.1997 Bulletin 1997/34**

(51) Int. Cl.⁶: **A61K 7/42**

(21) Numéro de dépôt: **95402440.2**

(22) Date de dépôt: **02.11.1995**

(54) **Compositions cosmétiques autobronzantes à base de dihydroacétone, d'alkylpolyosides et d'alcools gras**

Kosmetische Selbstbräunungsmittel auf der Basis von Dihydroxyaceton, Alkylpolyoxiden und Fettalkoholen

Self tanning cosmetic compositions containing dihydroxyacetone, alkylpolyosides and fatty alcohols

(84) Etats contractants désignés:
**AT BE CH DE ES FR GB IT LI NL SE**

(30) Priorité: **05.12.1994 FR 9414593**

(43) Date de publication de la demande:
**12.06.1996 Bulletin 1996/24**

(73) Titulaire: **L'OREAL**
**75008 Paris (FR)**

(72) Inventeur: **Hansenne, Isabelle**
**F-75017 Paris (FR)**

(74) Mandataire: **Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:
**EP-A- 0 382 619          WO-A-93/16683**

• **SEIFEN-öLE-FETTE-WACHSE, vol.98, no.24, 23 Novembre 1972, AUGSBURG page 828**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**Description**

La présente invention concerne de nouvelles compositions cosmétiques à usage topique plus particulièrement destinées au bronzage et/ou au brunissage artificiels de la peau (compositions ci-après dénommées plus simplement compositions autobronzantes), ainsi que leur utilisation dans l'application cosmétique susmentionnée. Plus précisément encore, elle concerne des compositions autobronzantes à activité d'une part et à stabilité d'autre part améliorées, qui comprennent, dans un support cosmétiquement acceptable, de la dihydroxyacétone à titre d'agent d'autobronzage, en association avec un ou plusieurs alkylpolyosides, en particulier des alkylpolyglucosides, et un ou plusieurs alcools gras.

On sait que la dihydroxyacétone, ou DHA, est un produit particulièrement intéressant qui est couramment utilisé en cosmétique comme agent de bronzage artificiel de la peau ; appliqué sur cette dernière, notamment sur le visage, il permet d'obtenir un effet de bronzage ou de brunissage d'apparence plus ou moins semblable à celui qui peut résulter d'une exposition prolongée au soleil (bronzage naturel) ou sous une lampe UV. Une telle utilisation présente en outre pour avantage d'éviter totalement les risques de réaction cutanée généralement attachés aux expositions prolongées précitées (érythèmes, brûlures, perte d'élasticité, apparition de rides, vieillissement prématuré de la peau, et autres).

L'un des problèmes rencontrés avec les compositions cosmétiques autobronzantes actuelles à base de DHA est que l'intensité de la coloration obtenue sur la peau et/ou sa tenue au cours du temps (résistance aux lavages notamment) et/ou la rapidité avec laquelle cette coloration se développe, sont souvent jugés comme insuffisants par les utilisateurs.

Par ailleurs, une autre difficulté réside dans le fait que la DHA présente une facheuse tendance, plus ou moins prononcée selon la nature du milieu dans lequel elle est formulée, à se dégrader au cours du temps, occasionnant par là des problèmes de stockage et/ou de conservation qui se traduisent généralement à terme par un jaunissement non souhaitable des compositions qui la contiennent.

Pour tenter d'obvier à ces divers inconvénients, il a déja été proposé, dans la demande de brevet FR-A- 2 698 267 au nom de la Demanderesse, d'associer la DHA avec un copolymère particulier agissant comme épaississant, à savoir un copolymère réticulé d'acrylamide/acide 2-acrylamido 2-méthylpropane sulfonique (dont le "SEPIGEL 305[®]" commercialisé par la Société SEPPIC).

Bien que de telles compositions contenant ce copolymère soient intéressantes et présentent effectivement des propriétés améliorées par rapport à celles qui en sont exemptes, elles ne donnent toutefois pas encore des résultats complètement satisfaisants.

La présente invention vise à proposer de nouvelles compositions cosmétiques à base de DHA qui présentent d'une part une efficacité et/ou une activité autobronzantes sur la peau améliorées (intensité et tenue), ainsi que, d'autre part, une excellente stabilité.

Ainsi, à la suite d'importantes recherches menées sur la question, il a maintenant été trouvé par la Demanderesse, et ceci de façon tout à fait inattendue et surprenante, qu'il est possible d'améliorer le pouvoir de coloration cutanée attaché aux compositions classiques de l'art antérieur à base de DHA, en introduisant conjointement dans ces dernières d'une part au moins composé choisi au sein des alkylpolyosides, en particulier des alkylpolyglucosides, et d'autre part au moins un composé choisi au sein des alcools gras, ces deux derniers composés pouvant par ailleurs être éventuellement associés à au moins un polyoside.

Toutes choses étant égales par ailleurs (notamment à concentration identique en DHA), on observe qu'une composition autobronzante conforme à l'invention, grâce à la présence d'un mélange de type alkylpolyoside/alcool gras, présente systématiquement, au niveau de son pouvoir et de sa qualité de coloration de la peau (intensité, durabilité au cours du temps et/ou face aux lavages), des propriétés améliorées par rapport à une même composition autobronzante ne contenant pas ce mélange particulier. Par ailleurs, il a été constaté de manière surprenante que, dans les compositions conformes à l'invention, la DHA présente une stabilité chimique nettement accrue (moindre décomposition au cours du temps), et ceci même à des températures relativement élevées.

Ces découvertes sont à la base de la présente invention.

Conformément à l'un des objets de la présente invention, il est donc maintenant proposé de nouvelles compositions cosmétiques plus particulièrement destinées au bronzage artificiel de la peau, du type comprenant, dans un support cosmétiquement acceptable, de la dihydroxyacétone à titre d'agent autobronzant, et qui sont essentiellement caractérisées par le fait qu'elles contiennent en outre au moins un alkylpolyoside, de préférence au moins un alkylpolyglucoside, et au moins un alcool gras.

Selon un mode particulier de réalisation de la présente invention, la composition autobronzante peut contenir en outre au moins un polyoside.

Selon un autre mode particulier de réalisation de la présente invention, l'association alkylpolyoside / alcool gras / (polyoside) qui est présente dans la composition autobronzante est mise en oeuvre sous la forme d'un mélange comprenant (% poids par rapport audit mélange) :

- de 10 à 80 % en poids d'alkylpolyoside(s), avantageusement d'alkylpolyglucoside(s),

- de 20 à 90 % en poids d'alcool(s) gras ayant avantageusement de 12 à 22 atomes de carbone, et plus préférentiellement encore de 12 à 18 atomes de carbone,

- et éventuellement de 0,5 à 5 % en poids d'un polyoside.

Par ailleurs, il est connu du document WO 93/16683 des émulsions pour colorer artifciellement la peau, stables, de pH de 2,5 à 7, comprenant, dans des proportions particulières, de la dihydroxyacétone, au moins un dérivé d'alkylhydroxyalkylcellulose et au moins un dérivé d'acide gras.

Dans le mélange ci-dessus, l'alkylpolyoside possède de préférence une partie alkyle identique à celle de l'alcool gras utilisé, et ce mélange correspond alors avantageusement au produit de réaction directement obtenu, en milieu acide, entre un ose et un excès stoechiométrique d'alcool gras, comme expliqué plus en détails par la suite.

D'autres caractéristiques, aspects et avantages de l'invention apparaitront à la lecture de la description détaillée qui va suivre.

La dihydroxyacétone ou DHA est présente dans les compositions conformes à l'invention dans des proportions suffisantes pour conférer à la peau, après application, une coloration similaire à celle obtenue à la suite d'un bronzage naturel. Cette DHA est ainsi généralement présente dans des proportions comprises entre 0,5 et 10 % en poids par rapport au poids total de la composition, et de préférence entre 1 et 6 % en poids.

Les alkylpolyosides susceptibles d'être mis en oeuvre dans le cadre de la présente invention sont des produits déja bien connus en soi, et largement utilisés à titre de tensio-actifs non ioniques dans une large gamme d'applications industrielles. Nombre de ces produits sont par ailleurs disponibles commercialement.

L'emploi de tels alkylpolyosides dans le but d'améliorer les propriétés des compositions cosmétiques autobronzantes à base de DHA n'a toutefois jamais été divulgué, préconisé ou même suggéré dans l'art antérieur.

Ces produits et leurs procédés de synthèse sont notamment décrits dans (1) "Hanbook of Surfactants", par M.R. PORTER, Editions Blackie & Son Ltd, 1991, pp 142-145 ; (2) "Alkylpolyglucosides : An Overview of the Patent Situation", par H. FABRY et al (HENKEL), revue HAPPI, Aôut 1994, pp 111-115 ; et (3) demande de brevet WO 92/06778 ; les documents (1) à (3) sont, mentionnés à titre de références dans la présente description.

D'une manière générale, ils peuvent être obtenus industriellement par réaction, en milieu acide (acides sulfurique, chlorhydrique, phosphorique...), entre un ose disposant d'un OH anomérique (glucose, dextrose...) et un alcool gras introduit en excès stochiométrique, puis éventuelle élimination par distillation de l'alcool gras non éthérifié, et enfin éventuelle élimination par filtration des produits provenant de l'éventuelle polycondensation des oses (polyosides). Le détail des conditions réactionnels à observer est notamment donné dans la demande WO 92/06778 précitée. Les compositions qui sont obtenues sans procéder à l'élimination (distillation) des alcools gras en excès, avec ou sans élimination subséquente des polyosides résiduels, correspondent ainsi aux mélanges alkylpolyoside / alcool gras / (polyoside) susceptibles d'être avantageusement utilisés dans le cadre de la présente invention, comme indiqué précédemment. Bien entendu, de tels mélanges peuvent également être obtenus par simple mélange physique des différents constituants présynthétisés.

Selon la présente invention, on utilise de préférence des alkylpolyosides choisis au sein de ceux présentant au moins l'une, et plus avantageusement encore l'ensemble, des caractéristiques suivantes :

- l'alkylpolyoside présente une partie alkyle, linéaire ou ramifiée, comprennant de 12 à 22 atomes de carbone, de préférence de 12 à 18 atomes de carbone, et encore plus préférentiellement de 16 à 18 atomes de carbone,

- l'alkylpolyoside comprend au moins un ose choisi dans le groupe constitué de glucose, dextrose, saccharose, fructose, galactose, maltose, maltotriose, lactose, cellobiose, mannose, ribose, dextrane, talose, allose, xylose, levoglucosane, cellulose et amidon , de préférence cet ose est choisi parmi glucose, dextrose, fructose et maltose ; encore plus préférentiellement, cet ose est constitué par glucose (alkylpolyglucosides),

- l'alkylpolyoside présente une chaine polyoside comprenant jusqu'à 30 unités, étant entendu que chaque unité de la partie polyoside peut être sous forme isomérique $\alpha$ ou $\beta$, sous forme L ou D, et la conformation du motif "ose" sous forme furanoside ou pyranoside avec un oxygène anomérique.

Selon la présente invention, il est bien entendu possible d'utiliser des mélanges d'alkylpolyosides, susceptibles de différer les uns des autres par la nature du motif alkyle porté et/ou la nature de la chaine polyoside porteuse.

Le ou les alkylpolyosides peuvent être présents dans les compositions autobronzantes conformes à l'invention à des teneurs généralement comprises entre 0,1 et 10 % en poids par rapport au poids total de la composition, de préférence à des teneurs comprises entre 2 et 8 %.

Concernant maintenant les alcools gras devant être utilisés, seuls ou en mélanges, en association avec les alkylpolyosides dans la préparation des compositions autobronzantes conformes à l'invention, il peut s'agir d'alcools gras linéaires ou ramifiés, d'origine synthétique, ou encore naturelle comme par exemple les alcools provenant de matières végétales (coprah, palmiste, palme...) ou animales (suif...). Bien entendu, d'autres alcools à longue chaine peuvent également être utilisés, comme par exemple les étheralcools ou bien encore les alcools dits de Guerbet. Enfin, on peut également utiliser certaines coupes plus ou moins longues d'alcools d'origine naturelle, comme par exemple coco ($C_{12}$ à $C_{16}$) ou suif ($C_{16}$ à $C_{18}$) ou des composés type diols ou cholesterol. Comme indiqué ci-dessus, on peut utiliser des mélanges d'alcools, tels que ceux pouvant provenir notamment du suif ou du coprah.

Selon un mode préféré de réalisation de la présente invention, le ou les alcools gras utilisés sont choisis au sein de ceux présentant de 12 à 22 atomes de carbone, et encore plus préférentiellement de 12 à 18 atomes de carbone.

A titre d'exemples particuliers d'alcools gras utilisables dans le cadre de la présente invention, on peut notamment citer l'alcool laurique, cétylique, myristique, stéarylique, palmitique et oléique, qui peuvent donc être pris seuls ou en mélanges

En outre, et comme indiqué précédemment, il est particulièrement avantageux, selon la présente invention, de mettre conjointement en oeuvre un alcool gras et un alkylpolyoside dont la partie alkyle est identique à celle de l'alcool gras retenu.

Le ou les alcools gras peuvent être présents dans les compositions autobronzantes conformes à l'invention à des teneurs généralement comprises entre 0,1 et 10 % en poids par rapport au poids total de la composition, de préférence à des teneurs comprises entre 0,4 et 9 % en poids.

Des produits commerciaux convenant à la mise en oeuvre de la présente invention sont notamment ceux vendus sous la dénomination de EMULGADE® PL 1618 par la Société HENKEL, ou ceux vendus sous la dénomination de MONTANOV 68® par la Société SEPPIC. Le produit référencé MONTANOV 68® convient particulièrement bien.

Les compositions autobronzantes conformes à l'invention peuvent se présenter sous forme de crèmes, de laits, de gels, de gel-crèmes, d'émulsions huile-dans-eau, de dispersions vésiculaires, de lotions fluides, en particulier de lotions fluides vaporisables, ou tout autre forme généralement utilisée en cosmétique, en particulier celle convenant habituellement aux compositions cosmétiques autobronzantes

Le milieu cosmétiquement acceptable (ou support) dans ces compositions peut classiquement contenir de l'eau, un mélange d'eau et d'un ou plusieurs solvants organiques, ou un solvant ou un mélange de solvants organiques cosmétiquement acceptables. Ce milieu contient également, sous une forme de réalisation préférentielle, des corps gras et/ou des silicones cosmétiquement acceptables.

Les solvants peuvent être plus particulièrement choisis parmi les alcools polyhydriques comme par exemple le glycérol, l'éthylèneglycol, le propylèneglycol, le diéthylèneglycol et le sorbitol, ou bien encore parmi les alcools inférieurs hydrosolubles tels que l'éthanol, l'isopropanol ou le butanol.

Les différents corps gras utilisables peuvent être, quant à eux, choisis, seuls ou en mélanges, parmi les huiles d'origine végétale, animale ou minérale, les cires naturelles ou synthétiques, et analogues.

Parmi les huiles pouvant rentrer dans la composition de la phase grasse, on peut ainsi notamment citer :

- les huiles minérales telles que l'huile de paraffine et l'huile de vaseline,

- les huiles d'origine animale telles que le perhydrosqualène,

- les huiles d'origine végétale telles que l'huile d'amande douce, l'huile d'avocat, l'huile de ricin, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore et l'huile de seigle,

- les huiles de synthèse telles que l'huile de purcellin, le myristate de butyle, le myristate d'isopropyle, le myristate de cétyle, le palmitate d'isopropyle, l'adipate d'isopropyle, l'adipate d'éthylhéxyle, le stéarate de butyle, le stéarate d'héxadécyle, le stéarate d'isopropyle, le stéarate d'octyle, le stéarate d'isocétyle, l'oléate de décyle, le laurate d'héxyle, le dicaprylate de propylène glycol et les esters dérivés d'acide lanolique tels que le lanolate d'isopropyle et le lanolate d'isocétyle, les isoparaffines et les poly-$\alpha$-oléfines.

Comme autres huiles utilisables, on peut encore citer les benzoates d'alcools gras en $C_{12}$-$C_{15}$ (Finsolv TN® de FINETEX), les acétylglycérides, les octanoates et décanoates d'alcools et de polyalcools tels que ceux de glycol et de glycérol, les ricinoléates d'alcools et de polyalcools tels que ceux de cétyle, les triglycérides d'acides gras tels que les triglycérides caprylique/caprique, les triglycérides d'acides gras saturés en $C_{10}$-$C_{18}$, les huiles fluorées et perfluorées, la lanoline, la lanoline hydrogénée, la lanoline acétylée, les huiles de silicones, volatiles ou non, ou encore les solutions organiques de gommes et/ou de résines d'organosiloxanes.

Bien entendu, les compositions selon l'invention peuvent également contenir un ou plusieurs adjuvants cosmétiques, lipophiles ou hydrophiles, classiques, notamment ceux qui sont déja utilisés de manière habituelle dans la fabrication et l'obtention des compositions cosmétiques autobronzantes.

Ainsi, parmi les adjuvants cosmétiques classiques susceptibles d'être contenus dans la phase aqueuse et/ou dans

4

la phase grasse des compositions conformes à l'invention (selon leur caractère hydro- et/ou liposoluble), on peut citer notamment les épaississants ioniques ou non ioniques, les adoucissants, les antioxydants, les opacifiants, les stabilisants, les émollients, les agents répulsifs contre les insectes, les filtres solaires organiques actifs dans l'UV-A et/ou l'UV-B, les pigments et les nanopigments minéraux photoprotecteurs, les agents hydratants, les vitamines, les parfums, les conservateurs, les charges, les séquestrants, les colorants, ou tout autre ingrédient habituellement utilisé dans le domaine des produits autobronzants.

Un autre objet de la présente invention est constitué par l'utilisation des compositions telles que ci-dessus définies comme, ou pour la fabrication de, compositions cosmétiques pour le bronzage et/ou le brunissage artificiels de la peau. Comme indiqué précédemment, les compositions peuvent alors être conditionnées sous la forme de crèmes, de laits, de gels crèmes, ou bien encore de lotions fluides, en particulier de lotions fluides vaporisables, ou tout autre forme appropriée.

Un autre objet de la présente invention réside dans un procédé de traitement cosmétique de la peau destiné à la bronzer et/ou la brunir artificiellement et qui consiste essentiellement à appliquer sur celle-ci une quantité efficace d'une composition cosmétique telle que définie ci-dessus. Un exemple concret, destiné à illustrer l'invention va maintenant être donné.

EXEMPLE

Dans cet exemple, on a préparé et comparé deux compositions autobronzantes concrètes à base de dihydroxyacétone (5% poids), l'une étant conforme à l'invention (C1) et contenant un mélange alkylpolyoside/alcool gras ("MONTANOV 68[®]" de chez SEPPIC), l'autre comparative (C2) ne se différenciant de C1 que par le fait qu'elle contenait à la place dudit mélange un copolymère réticulé d'acrylamide/acide 2-acrylamido 2-méthylpropane sulfonique tel que décrit dans FR-A- 2 698 267 ("SEPIGEL 305[®]" de chez Seppic), cette composition comparative étant en effet considérée comme la composition la plus performante de l'art antérieur.

Les compositions chimiques (% en poids ramenés à l'ensemble de la formulation) de ces deux formulations étaient ainsi les suivantes :

| Phase A : | |
|---|---|
| - Huile de silicone | 10% |
| - Benzoate d'alcools en $C_{12}$-$C_{15}$ ("FINSOLV TN[®]" de chez WITCO) | 5 % |
| - 4-(ter.-butyl) 4'-méthoxy dibenzoylméthane (filtre solaire "PARSOL 1789[®]" de chez GIVAUDAN) | 0,5 % |
| - 4-méthylbenzylidène camphre (filtre solaire "EUSOLEX 6300[®]" de chez MERCK) | 1,5% |
| - MONTANOV 68[®] (*) (pour la composition C1) ou | 7,5% |
| SEPIGEL 305[®] (pour la compositon C2) | 3 % |
| Phase B : | |
| - Dihydroxyacétone | 5 % |
| - Eau | 30 % |
| Phase C : | |
| - Glycérol | 3 % |
| - Sorbitol en solution aqueuse à 70% | 2 % |
| - Eau                              qsp | 100% |
| Phase D : | |
| - Parfum | qs |
| - Conservateurs | qs |

(*) : le MONTANOV 68[®] est un produit commercial dont la composition s'avère correspondre à celle du produit de rèaction dècrit à l'exemple 1 de la demande de brevet WO 92/06778 au nom de la Sociètè SEPPIC

Le mode opératoire qui a été suivi pour préparer ces compositions a été le suivant : les phases grasse (A) et aqueuse (C) ont été toutes deux préalablement portées à une température de l'ordre de 90°C ; puis on a ajouté la phase aqueuse *(C)* dans la phase grasse *(A),* et ceci sous agitation énergique de cette dernière au moyen d'une turbine type MORITZ (1000 t/mn) ; et enfin, on a introduit dans l'émulsion résultante, vèrs 40 °C, d'abord la phase *(B)* puis la phase *(D).*

On a ensuite comparé le pouvoir colorant sur peau des deux compositions C1 et C2 ainsi obtenues. Le pouvoir de coloration a été apprécié au moyen du test suivant : on a appliqué les compositions, à raison de 2 mg/cm$^2$ de peau (carrés de 6 cm$^2$ environ), sur les avant-bras de trois personnes témoins (P1, P2 et P3) et on a mesuré sur ces avant-bras l'écart colorimétrique de la valeur L (coordonnée chromatique de luminance, mesurée à l'aide d'un colorimètre MINOLTA CM 1000) avant (To) et après (5 heures et 24 heures) application, de manière à déterminer une valeur absolue moyenne ΔL qui rend compte de l'intensité de la coloration obtenue sur la peau après application (plus le ΔL moyen est élevé, plus la coloration est intense) :

$$\Delta L(5H) = L_{To} - L_{T5h}$$

$$\Delta L(24\,H) = L_{To} - L_{T24h}$$

De manière à également quantifier la rémanence à l'eau des colorations obtenues, un lavage au savon a été réalisé sur les avant-bras traités de chaque témoin, ce lavage intervenant après la mesure à $T_{5h}$ et avant la mesure à $T_{24h}$.

Les résultats obtenus sont rassemblés dans le tableau I donné ci-après. Ces résultats montrent clairement que la composition C1 conforme à l'invention apporte sur la peau une coloration significativement plus intense que la composition comparative C2, tant 5 heures que 24 heures après l'application, et ceci même après lavage intermédiaire.

Par ailleurs, pour les deux compositions ci-dessus, la stabilité de la DHA après une conservation de 2 mois à température ambiante d'une part et à 45 °C d'autre part, a été quantifiée, et ceci au moyen d'une analyse par HPLC réalisée sur colonne MERCK LICHROSPHER SI 100 (Eluant : mélange éthanol(5%)/Cl2CH2(95%) en volumes ; pression 750 psi) permettant ainsi de suivre au cours du temps le taux de DHA non dégradée.

Les résultats obtenus ont également été rassemblés dans le tableau I ci-dessous.

Ces résultats montrent clairement que la DHA se conserve mieux à 45 °C dans la composition conforme à l'invention que dans la composition comparative.

TABLEAU I

| COMPOSITION | COLORATION (ΔL)[1] | | TAUX DE CONSERVATION DE LA DHA (%) | |
|---|---|---|---|---|
| | après 5 H | après 24 H | après 2 mois a T° ambiante | après 2 mois à 45°C |
| **C1** (invention) | 3,2 | 3,45 | 100 | 89 |
| **C2** (comparative) | 2,9 | 2,6 | 100 | 73 |

[1]:moyenne

## Revendications

1. Compositions cosmétiques à usage topique utilisables pour le bronzage et/ou le brunissage artificiels de la peau, comprenant dans un support cosmétiquement acceptable de la dihydroxyacétone, caractérisées par le fait qu'elles contiennent en outre au moins un alkylpolyoside et au moins un alcool gras, ainsi qu'éventuellement au moins un polyoside.

2. Compositions selon la revendication 1, caractérisées par le fait le ou les alkylpolyosides représentent de 10 à 80 % du poids total du mélange alkylpolyoside(s)/alcool(s) gras/(polyoside(s)), le ou les alcools gras de 20 à 90 % de ce poids total, et le ou les éventuels polyosides de 0,5 à 5 % de ce poids total.

3. Compositions selon l'une quelconque des revendications 1 ou 2, caractérisées par le fait que le ou les alkylpolyosides sont présents à une teneur comprise entre 0,1 et 10 % en poids par rapport au poids total de la composition.

4. Compositions selon la revendication 3, caractérisées par le fait que ladite teneur est comprise entre 2 et 8 %.

5. Compositions selon l'une quelconque des revendications précédentes, caractérisées par le fait que le ou les alcools gras sont présents à une teneur comprise entre 0,1 et 10 % en poids par rapport au poids total de la composition.

6. Compositions selon la revendication 5, caractérisées par le fait que ladite teneur est comprise entre 0,4 et 9 %.

7. Compositions selon l'une quelconque des revendications précédentes, caractérisées par le fait que la dihydroxyacétone est présente à une teneur comprise entre 0,5 et 10 % en poids par rapport au poids total de la composition.

8. Compositions selon la revendication 7, caractérisées par le fait que ladite teneur est comprise entre 1 et 6 %.

9. Compositions selon l'une quelconque des revendications précédentes, caractérisées par le fait que le ou les alkylpolyosides présentent une partie alkyle comprennant de 12 à 22 atomes de carbone.

10. Compositions selon la rendication 9, caractérisées par le fait que ladite partie alkyle comprend de 12 à 18 atomes de carbone.

11. Compositions selon la revendication 10, caractérisées par le fait que ladite partie alkyle comprend de 16 à 18 atomes de carbone.

12. Compositions selon l'une quelconque des revendications précédentes, caractérisées par le fait le ou les alkylpolyosides comprennent au moins un ose choisi dans le groupe constitué par glucose, dextrose, saccharose, fructose, galactose, maltose, maltotriose, lactose, cellobiose, mannose, ribose, dextrane, talose, allose, xylose, levoglucosane, cellulose et amidon.

13. Compositions selon la revendication 12, caractérisées par le fait que l'ose est choisi parmi glucose, dextrose, fructose et maltose.

14. Compositions selon la revendication 13, caractérisées par le fait que l'ose est le glucose.

15. Compositions selon l'une quelconque des revendications précédentes, caractérisées par le fait que le ou les alcools gras sont choisis au sein de ceux présentant de 12 à 22 atomes de carbone.

16. Compositions selon la revendication 15, caractérisées par le fait que lesdits alcools gras présentent de 12 à 18 atomes de carbone.

17. Compositions selon l'une quelconque des revendications précédentes, caractérisées par le fait que le ou les alkylpolyosides présentent une partie alkyle identique à celle du ou des alcools gras.

18. Utilisation des compositions définies à l'une quelconque des revendications 1 à 17 comme, ou pour la fabrication de, compositions cosmétiques destinées au bronzage et/ou au brunissage artificiels de la peau.

19. Procédé de traitement cosmétique pour bronzer et/ou brunir artificiellement la peau, caractérisé par le fait qu'il consiste à appliquer sur celle-ci une quantité efficace d'une composition telle que définie à l'une quelconque des revendications 1 à 17.

20. Utilisation d'un mélange entre au moins un alkylpolyoside et au moins un alcool gras tels que définis à l'une quelconque des revendications 1 à 17 pour améliorer le pouvoir colorant et/ou la stabilité d'une dihydroxyacétone contenue dans une composition cosmétique, en particulier une composition cosmétique destinée au bronzage et/ou au brunissage artificiels de la peau.

**Claims**

1. Cosmetic compositions for topical use which can be used for the artificial tanning and/or darkening of the skin, comprising, in a cosmetically acceptable carrier, dihydroxyacetone, characterized in that they contain, in addition, at least one alkylpolysaccharide and at least one fatty alcohol, and optionally at least one polysaccharide.

2. Compositions according to Claim 1, characterized in that the alkylpolysaccharide(s) represent from 10 to 80 % of the total weight of the alkylpolysaccharide(s)/fatty alcohol(s)/(polysaccharide(s)) mixture, the fatty alcohol(s) from 20 to 90 % of this total weight, and the optional polysaccharide(s) from 0.5 to 5 % of this total weight.

3. Compositions according to either of Claims 1 and 2, characterized in that the alkylpolysaccharides are present in an amount of between 0.1 and 10 % by weight relative to the total weight of the composition.

4. Compositions according to Claim 3, characterized in that the said amount is between 2 and 8 %.

5. Compositions according to any one of the preceding claims, characterized in that the fatty alcohol(s) are present in an amount of between 0.1 and 10 % by weight relative to the total weight of the composition.

6. Compositions according to Claim 5, characterized in that the said amount is between 0.4 and 9 %.

7. Compositions according to any one of the preceding claims, characterized in that the dihydroxyacetone is present in an amount of between 0.5 and 10 % by weight relative to the total weight of the composition.

8. Compositions according to Claim 7, characterized in that the said amount is between 1 and 6 %.

9. Compositions according to any one of the preceding claims, characterized in that the alkylpolysaccharide(s) have an alkyl portion comprising from 12 to 22 carbon atoms.

10. Compositions according to Claim 9, characterized in that the said alkyl portion comprises from 12 to 18 carbon atoms.

11. Compositions according to Claim 10, characterized in that the said alkyl portion comprises from 16 to 18 carbon atoms.

12. Compositions according to any one of the preceding claims, characterized in that the alkylpolysaccharides comprise at least one saccharide chosen from the group consisting of glucose, dextrose, sucrose, fructose, galactose, maltose, maltotriose, lactose, cellobiose, mannose, ribose, dextran, talose, allose, xylose, levoglucosan, cellulose and starch.

13. Compositions according to Claim 12, characterized in that the saccharide is chosen from glucose, dextrose, fructose and maltose.

14. Compositions according to Claim 13, characterized in that the saccharide is glucose.

15. Compositions according to any one of the preceding claims, characterized in that the fatty alcohol(s) are chosen from those having from 12 to 22 carbon atoms.

16. Compositions according to Claim 15, characterized in that the said fatty alcohols have from 12 to 18 carbon atoms.

17. Compositions according to any one of the preceding claims, characterized in that the alkylpolysaccharide(s) have an alkyl portion which is identical to that of the fatty alcohol(s).

18. Use of the compositions defined in any one of Claims 1 to 17 as, or for the manufacture of, cosmetic compositions intended for the artificial tanning and/or darkening of the skin.

19. Process of cosmetic treatment for artificially tanning and/or darkening the skin, characterized in that it consists in applying thereto an effective quantity of a composition as defined in any one of Claims 1 to 17.

20. Use of a mixture between at least one alkylpolysaccharide and at least one fatty alcohol as defined in any one of Claims 1 to 17 for improving the colouring power and/or the stability of a dihydroxyacetone contained in a cosmetic composition, in particular a cosmetic composition intended for the artificial tanning and/or darkening of the skin.

**Patentansprüche**

1. Kosmetische Zusammensetzungen zur topischen Anwendung, die zur künstlichen Bräunung und/oder Braunfärbung der Haut verwendet werden können und in einem kosmetisch akzeptablen Träger Dihydroxyaceton enthalten, dadurch gekennzeichnet, daß sie ferner mindestens ein Alkylpolyglykosid, mindestens einen Fettalkohol sowie gegebenenfalls mindestens einen Polyzucker enthalten.

2. Zusammensetzungen nach Anspruch 1, dadurch gekennzeichnet, daß das oder die Alkylpolyglykoside 10 bis 80 % des Gesamtgewichts des Gemisches Alkylpolyglykosid(e)/Fettalkohol(e)/Polyzucker, der oder die Fettalkohole 20 bis 90 % des Gesamtgewichts und der oder die gegebenenfalls vorliegenden Polyzucker 0,5 bis 5 % des Gesamtgewichts ausmachen.

3. Zusammensetzungen nach einem der Ansprüche 1 oder 2, dadurch gekennzeichnet, daß das oder die Alkypolyglykoside in einem Mengenanteil von 0,1 bis 10 Gew.-%, bezogen auf das Gesamtgewicht der Zusammensetzung, enthalten sind.

4. Zusammensetzungen nach Anspruch 3, dadurch gekennzeichnet, daß der Mengenanteil im Bereich von 2 bis 8 Gew.-% liegt.

5. Zusammensetzungen nach einem der vorhergehenden Ansprüche, dadurch gekennzeichnet, daß der oder die Fettalkohole in einem Mengenanteil von 0,1 bis 10 Gew.-%, bezogen auf das Gesamtgewicht der Zusammensetzung, enthalten sind.

6. Zusammensetzungen nach Anspruch 5, dadurch gekennzeichnet, daß der Mengenanteil im Bereich von 0,4 bis 9 Gew.-% liegt.

7. Zusammensetzungen nach einem der vorhergehenden Ansprüche, dadurch gekennzeichnet, daß das Dihydroxyaceton in einem Mengenanteil von 0,5 bis 10 Gew.-%, bezogen auf das Gesamtgewicht der Zusammensetzung, enthalten ist.

8. Zusammensetzungen nach Anspruch 7, dadurch gekennzeichnet, daß der Mengenanteil im Bereich von 1 bis 6 Gew.-% liegt.

9. Zusammensetzungen nach einem der vorhergehenden Ansprüche, dadurch gekennzeichnet, daß das oder die Alkylpolyglykoside einen Alkylanteil mit 12 bis 22 Kohlenstoffatomen aufweisen.

10. Zusammensetzungen nach Anspruch 9, dadurch gekennzeichnet, daß der Alkylanteil 12 bis 18 Kohlenstoffatome enthält.

11. Zusammensetzungen nach Anspruch 10, dadurch gekennzeichnet, daß der Alkylanteil 16 bis 18 Kohlenstoffatome enthält.

12. Zusammensetzungen nach einem der vorhergehenden Ansprüche, dadurch gekennzeichnet, daß das oder die Alkylpolyglykoside mindestens einen Zucker enthalten, der unter Glucose, Dextrose, Saccharose, Fructose, Galactose, Maltose, Maltotriose, Lactose, Cellubiose, Mannose, Ribose, Dextran, Talose, Allose, Xylose, Levoglucosan, Cellulose und Stärke ausgewählt ist.

13. Zusammensetzungen nach Anspruch 12, dadurch gekennzeichnet, daß der Zucker unter Glucose, Dextrose, Fructose und Maltose ausgewählt ist.

14. Zusammensetzungen nach Anspruch 13, dadurch gekennzeichnet, daß der Zucker Glucose ist.

15. Zusammensetzungen nach einem der vorhergehenden Ansprüche, dadurch gekennzeichnet, daß der oder die Fettalkohole unter den Alkoholen mit 12 bis 22 Kohlenstoffatomen ausgewählt sind.

16. Zusammensetzungen nach Anspruch 15, dadurch gekennzeichnet, daß die Fettalkohole 12 bis 18 Kohlenstoffatome aufweisen.

17. Zusammensetzungen nach einem der vorhergehenden Ansprüche, dadurch gekennzeichnet, daß das oder die Alkylpolyglykoside einen Alkylanteil aufweisen, der mit der Alkylgruppe des oder der Fettalkohole identisch ist.

18. Verwendung der Zusammensetzungen nach einem der Ansprüche 1 bis 17 als kosmetische Zusammensetzungen zur künstlichen Bräunung und/oder Braunfärbung der Haut oder zu deren Herstellung.

19. Verfahren zur kosmetischen Behandlung zur künstlichen Bräunung und/oder Braunfärbung der Haut, dadurch gekennzeichnet, daß es darin besteht, eine Zusammensetzung nach einem der Ansprüche 1 bis 17 auf die Haut aufzutragen.

20. Verwendung eines Gemisches aus mindestens einem Alkylpolyglykosid und mindestens einem Fettalkohol nach einem der Ansprüche 1 bis 17 zur Verbesserung des Hautfärbevermögens und /oder der Stabilität von Dihydroxyaceton, das in einer kosmetischen Zusammensetzung und insbesondere einer kosmetischen Zusammensetzung zur künstlichen Bräunung und/oder Braunfärbung der Haut enthalten ist.